(19) Europäisches Patentamt
European Patent Office
Office européen des brevets

(11) Veröffentlichungsnummer: **0 268 548 B1**

(12) **EUROPÄISCHE PATENTSCHRIFT**

(45) Veröffentlichungstag der Patentschrift: **21.08.91**

(51) Int. Cl.[5]: **C07C 405/00**, C07F 7/18, C07D 317/72, C07D 319/08, C07C 69/66

(21) Anmeldenummer: **87730147.3**

(22) Anmeldetag: **12.11.87**

# (54) Verbesserte Ketoreduktion von Carbacyclinzwischenprodukten.

(30) Priorität: **13.11.86 DE 3638761**

(43) Veröffentlichungstag der Anmeldung:
**25.05.88 Patentblatt 88/21**

(45) Bekanntmachung des Hinweises auf die Patenterteilung:
**21.08.91 Patentblatt 91/34**

(84) Benannte Vertragsstaaten:
**AT BE CH DE ES FR GB GR IT LI LU NL SE**

(56) Entgegenhaltungen:
**EP-A- 0 136 779**
**GB-A- 2 148 293**

**JOURNAL OF THE AMERICAN CHEMICAL SOCIETY, Band 103, Nr. 18, 9. September 1981, Seiten 5454-5459, American Chemical Society, Gaston, Pa., US; A.L. GEMAL et al.: "Lanthanoids in organic synthesis. 6. The reduction of alpha-enones by sodium borohydride in the presence of lanthanoid chlorides: synthetic and mechanistic aspects"**

**CHEMICAL ABSTRACTS, Band 94, Nr. 21, 25. Mai 1981, Seite 695, Zusammenfassung Nr. 174751b, Columbus, Ohio, US; G. RUECKER et al.: "Stereoselective reduction of cyclic 2,3-epoxyketones to trans-2,3-epoxy alcohols", & SYNTH. COMMUN. 1980, 10(8), 623-6**

(73) Patentinhaber: **SCHERING AKTIENGESELLSCHAFT Berlin und Bergkamen**
**Müllerstrasse 170/178 Postfach 65 03 11**
**W-1000 Berlin 65(DE)**

(72) Erfinder: **Dahl, Helmut, Dr.**
**Gollanczstrasse 102**
**W-1000 Berlin 28(DE)**
Erfinder: **Büttner, Gabriela**
**Steinmetzstrasse 21**
**W-1000 Berlin 30(DE)**
Erfinder: **Peschel, Dieter**
**Lütticher Strasse 49**
**W-1000 Berlin 65(DE)**

**EP 0 268 548 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein neues Verfahren zur Reduktion von 15-Ketocarbacyclin-Zwischenprodukten (PG-Nomenklatur)in Gegenwart von Cer-III-Salzen.

Bei den Synthesen der pharmakologisch wirksamen Carbacyclin-Analogen Iloprost, Cicaprost bzw. Eptaprost ist die Reduktion der 15-Ketogruppe zur 15α-Hydroxygruppe eine sehr wichtige Stufe. Die Reduktion mit technisch leicht zugänglichen Reagenzien wie Natriumborhydrid führt zu einem Gemisch mit dem unerwünschten 15β-Hydroxy-Isomeren. Die beiden Isomeren müssen durch Chromatographie voneinander getrennt werden. (Aus wirtschaftlichen Gründen muß das 15β-Isomere zum Ausgangsketon zurückoxidiert und erneut reduziert und in die 15-Isomeren getrennt werden usw.). Der zur Trennung erforderliche Aufwand (Adsorbens, Lösungsmittelmenge) ist um so höher, je mehr 15β-Hydroxy-Isomeres abgetrennt werden muß.

EP-136 779 beschreibt die Verwendung eines Ce-III Salzes in einem Verfahren zur Herstellung von Carbacyclin-Analogen. Nach Seite 93 ersten Absatz von EP-136779 dient der Zusatz von Cerchlorid lediglich der Minimisierung der Reduktion von C-C-Doppelbindungen.

Nach den bisherigen Verfahren ist der Anteil an unerwünschten 15β-Hydroxy-Isomeren stets hoch, soweit es sich um die Reduktion mit einfachen Hydrid-Reagenzien handelt.

Zudem wird Iloprost, das ein Diastereomeren-Gemisch der 16-Methylverbindungen im Verhältnis 16α:16β = 54:46 darstellt, über diesen Weg bisher nur erhalten, wenn die oben erwähnte Rückoxidation ein- bis zweimal durchgeführt wird und alle nach Reduktion und Chromatographie erhaltenen 15α-Hydroxyprodukte (die für sich unterschiedliche Diastereomeren-Zusammensetzungen aufweisen) vereint weiterverarbeitet werden. Die chemische Reduktion erfordert, wie man unschwer erkennen kann, einen außerordentlich hohen Aufwand.

Bei der Iloprost-Synthese kann die 15-Ketogruppe (3a, Schema 1) auch mikrobiologisch reduziert werden (zu 4a). Der gesamte Aufwand der Durchführung einer mikrobiologischen Reduktion, der Aufarbeitung und der Aufreinigung des Produkts bis zur Abtrennung unerwünschter Begleitstoffe ist jedoch sehr aufwendig.

Bei der Cicaprost- und Eptaprost-Zwischenstufe 1 ist die mikrobiologische Reduktion nicht durchführbar.

Sie läßt sich auch bei 3b nicht durchführen. 3b ist durch die gleichzeitige angemeldeten Synthesen über 3α-Hydroxy-cis-bicyclo[3.3.0]octan-7-on-2β-carbonsäuremethylester-Derivate, insbesondere das 7,7-Neopentylketal, auf wesentlich einfachere Weise als die bisherige Vorstufe 3a herstellbar.

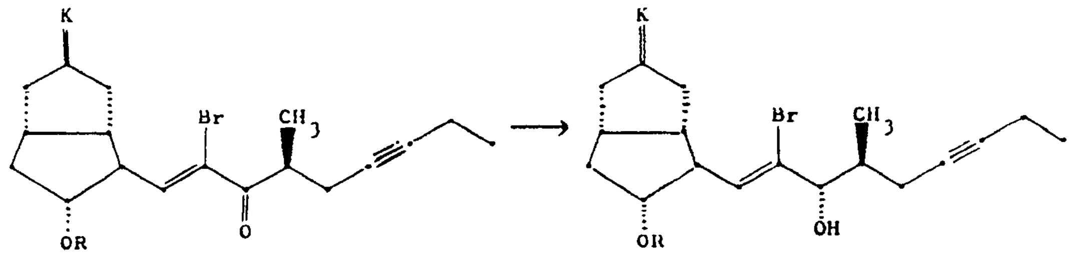

1 a: K = $-O-CH_2-CH_2-O-$

b: K = $-O-CH_2-C(CH_3)_2-CH_2-O-$

c: K = $-O-CH_2-C(CH_3)_2-CH_2-O-$

R = $-CO-C_6H_5$

2

R = -CO- 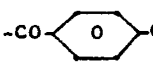 $-C_6H_5$

R = $-Si(CH_3)_2[C(CH_3)_3]$

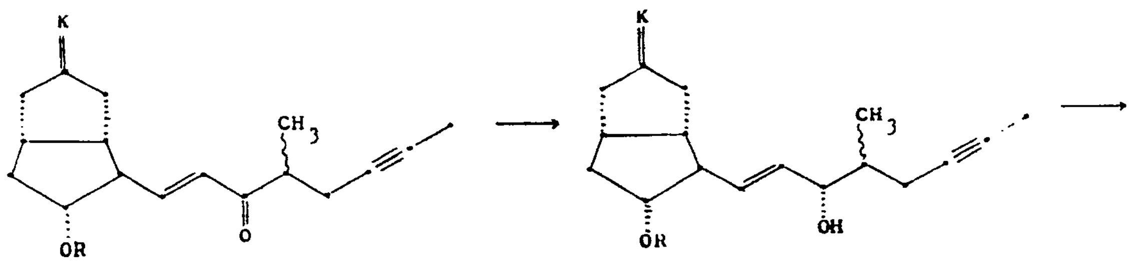

3 a-c

4 a-c

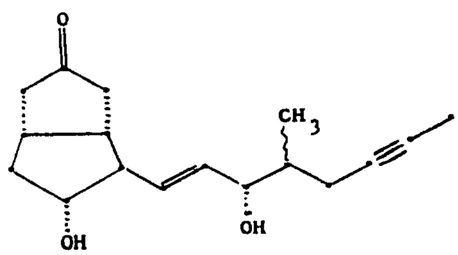

5

a) K = $-O-CH_2-CH_2-O-$

b) K = $-O-CH_2-C(CH_3)_2-CH_2-O$

c) K = $-O-CH_2-C(CH_3)_2-CH_2-O-$

d) K = $-O-CH_2-C(CH_3)_2-CH_2-O-$

e) K = $-O-CH_2-C(CH_3)_2-CH_2-O-$

R = $-CO-C_6H_5$

R = $-Si(CH_3)_2C(CH_3)_3$

R = -O 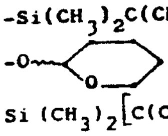

R = $Si(CH_3)_2[C(CH_3)_2CH(CH_3)_2]$

R = $Si(C_6H_5)_2[C(CH_3)_3]$

Das aus den THP-ether-Vorstufen (an Stelle der erwähnten Silylether) erhältliche Keton 3c führt bei der Natriumborhydrid-Reduktion zu weniger guten Ausbeuten und zu einer unbefriedigenden 16-Diastereomeren-Verteilung. Zudem lassen sich die 15-Isomeren erst nach Abspaltung der Schutzgruppen trennen, was die Verwertbarkeit des 15β-Anteils durch Rückoxidation erschwert. Der Weg über 3c ist daher ungünstiger als über 3b.

Es bestand daher die Aufgabe, die chemische Reduktion der 15-Ketogruppe bei der Synthese von Carbacyclin-Analoga bezüglich der Ausbeute an 15α-Hydroxy-Produkt und bei der Iloprost-Synthese auch hinsichtlich der 16-Diastereomeren-Zusammensetzung zu verbessern.

Die Erfindung betrifft somit ein Verfahren zur Herstellung von α-Hydroxy-bicyclo[3.3.0]octan-Derivaten der Formel I

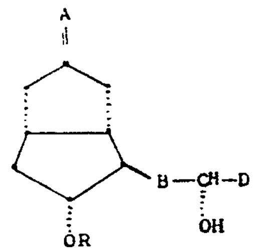

(I),

worin

A den 2bindigen Rest -O-X-O- mit X als gerad- oder verzweigt kettigem Alkylen mit 1 - 7 C-Atomen oder die Reste $=CH-(CH_2)_3-COOR'$, $=CH-CH_2-O-CH_2-COOR'$ oder $=CH-(CH_2)_3-O-CH_2-CH_2-COOR'$ mit R' als $C_1$-$C_7$-Alkyl bedeutet,

R den Rest

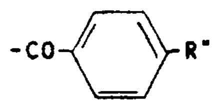

mit R" als Wasserstoff oder Phenyl oder den Rest $-SiR_1R_2R_3$, wobei $R_1$,$R_2$ und $R_3$ gleich oder verschieden sein können und eine gerad- oder verzweigtkettige Alkylgruppe mit 1 - 7 C-Atomen oder Phenyl darstellen,

B eine trans-CH = C(X)-Gruppe mit X als Wasserstoff oder Brom, wobei sich die trans-Konfiguration auf die C-Kette bezieht, und

D eine Alkylgruppe mit 1 - 10 C-Atomen, eine Alkenylgruppe mit 2 - 10 C-Atomen oder eine Alkinylgruppe mit 2 -10 C-Atomen bedeuten,

dadurch gekennzeichnet, daß man Keto-bicyclo[3.3.0]octan-Derivate der Formel II

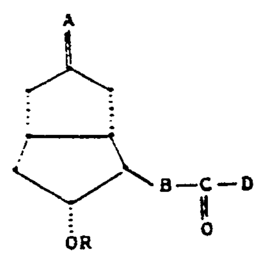

(II),

in Gegenwart von Cer-III-Salzen reduziert.

Bei der Lösung der Aufgabe sind zwei Effekte wichtig, die einzeln, besonders aber miteinander kombiniert, vorteilhaft sind:

a) Durchführung der Reduktion mit Natriumborhydrid in Gegenwart von Cer-3-chlorid führt zu einer deutlichen Ausbeutesteigerung an gewünschtem 15α-Hydroxy-produkt:

| Reaktion | Reduktionsmittel | 15α-OH: 15β-OH | Ausbeute-erhöhung | Beispiel |
|---|---|---|---|---|
| 1a → 2a | $NaBH_4$ | 40 : 60 | 35 % | 1b |
| | $NaBH_4$ / $CeCl_3$ | 54 : 46 | | 1a |
| 1b → 2b | $NaBH_4$ | 46 : 54 | 22 % | 2b |
| | $NaBH_4$ / $CeCl_3$ | 56 : 44 | | 2a |
| 1c → 2c | $NaBH_4$ / $CeCl_3$ | 89 : 11 | | 4 |

Die Ausbeuteerhöhung beträgt somit mehr als 100 %, wenn man den Schutzgruppeneffekt mit berücksichtigt.
Die Herstellung von 1c erfolgt in Analogie zur Herstellung von 1a aus dem im Beispiel A 1 beschriebenen Carbaldehyd.
Zur Verwendung der erhaltenen Verbindung 2c eignen sich die gleichen Verfahren, die zur Verwendung der Verbindungen 2a und 2b. geeignet sind. Die Abspaltung der Schutzgruppen erfolgt in Analogie zum Beispiel A 3.
b) Schutzgruppeneinflüsse
Hierzu zählt besonders der Ersatz der bisher eingesetzten 11-Ester durch 11-Silylether.

| Reaktion | Reduktions-mittel | 15α-OH:15β-OH | 16α-$CH_3$:16β-$CH_3$ | Ausbeute-erhöhung | Beispiel |
|---|---|---|---|---|---|
| 3a → 4a | $NaBH_4$ | 55:45 | 60:40 | | |
| | $NaBH_4$/$CeCl_3$ | 55:45 | 60:40 | 60 % | |
| 3b → 4b | $NaBH_4$/$CeCl_3$ | 89:11 | 54:46 | | 3a |
| | $NaBH_4$ | 74:26 | 59:41 | | 3b |
| 3c → 4c | $NaBH_4$/$CeCl_3$ | 67:33 | 57:43 | | |

Die hohe Ausbeute bei der erfindungsgemäßen Reduktion von 3b zu 4b hat zur Folge, daß auch die 16-Methyl-Diastereomeren-Verteilung richtig ist, womit die Rückoxidation des 15β-OH-Anteils entfallen kann, die Chromatographie erleichtert wird und ein gut zugängliches Ausgangsmaterial für die Synthese von

Iloprost verwendet werden kann.

(+)-3α-Hydroxy-7,7-(2,2-dimethyl-trimethylendioxy)-cis-bicyclo[3.3.0]octan-2β-carbonsäure-methylester, hergestellt z.B. nach der deutschen Anmeldung P 36 38 758.4, wird nach den von H. Wetter und K. Oertle. Tetrahedron Letters 26, 5515 (1985) bzw. von S. Hanessian und P. Lavallee, Can. J. Chem. 53, 2975 (1975) angegebenen Verfahren in die Silylether überführt und die Carbonsäure-methylester-Gruppe in Analogie zu den von K. Mori und M. Tsuji. Tetrahedron 42, 435 (1986) beschriebenen Bedingungen, jedoch bis -40° C, reduziert. Die weitere Umsetzung erfolgt wie in den Beispielen A 1 und A 2 beschrieben. Als Silylchloride werden Thexyl-dimethyl-chlorsilan und tert.-Butyl-diphenyl-chlorsilan eingesetzt.

Die Schutzgruppenabspaltung aus der erhaltenen Verbindung 4d erfolgt in Analogie zum Beispiel A 3. Bei der Verbindung 4e wird die Schutzgruppenabspaltung im allgemeinen nacheinander erfolgen. Der Silylether wird z.B. mit Tetrabutylammoniumfluorid in Tetrahydrofuran gespalten und das Ketal unter den Bedingungen des Beispiels A 3. Die Schritte können vertauscht werden. In allen Fällen entsteht die Verbindung 5.

Der Zusatz von Cer-3-chlorid bei der Reduktion von 15-Keto-prostaglandin Zwischenprodukten ist bekannt (J.-L. Luche, J. Amer. Chem. Soc. 100, 2226 (1978), J.C.S. Chem. Comm. 1978, 601). Er dient jedoch dazu, unerwünschte 1,4-Additionen, d.h. die Reduktion der $C_{13}$-$C_{14}$-Doppelbindung, zu vermeiden. Über eine Verbesserung des 15α/β-Verhältnisses in den Produkten ist bisher nichts bekannt. Man findet im Gegenteil bei der Anwendung dieser Arbeitstechnik häufig keine Veränderung eher eine Verschlechterung der Ausbeute an 15α-Hydroxy-Produkten, wie in der folgenden Übersicht an zwei Beispielen dargestellt wird.

| Edukt | Reduktionsmittel | 15α/15β-OH |
|---|---|---|
| 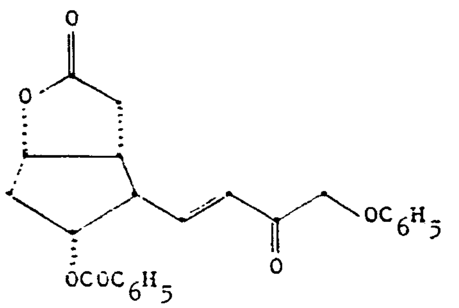 | $NaBH_4$ | 50:50 |
| | $NaBH_4/CeCl_3$ | 45:55 |
| 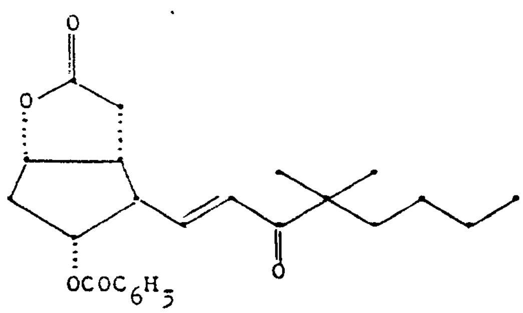 | $NaBH_4$ | 50:50 |
| | $NaBH_4/CeCl_3$ | 50:50 |

Es ist daher überraschend, daß Cer-III-chlorid den 15α-Hydroxy-Anteil bei der Reduktion von Verbindungen der Formel II deutlich steigert.

Bevorzugt werden Ausgangsmaterialien, die sich aus gut zugänglichen Carbacyclin-Vorstufen unter Verwendung handelsüblicher und ausreichend stabiler Schutzgruppen herstellen lassen. Die Anwendung der erfindungsgemäßen Reaktion soll durch diese Auswahl jedoch nicht eingeschränkt werden.

Natriumborhydrid und Cer-3-chlorid werden deshalb bevorzugt verwendet, weil sie leicht in großer Menge erhältlich sind. Die Reduktion ließe sich jedoch auch mit anderen Borhydriden und anderen Cer-3-Salzen durchführen, soweit sich diese unter den Reaktionsbedingungen nicht zersetzen.

Natriumborhydrid muß in mindestens stöchiometrischen Mengen eingesetzt, Cer-3-Salze können dagegen auch auf katalytische Mengen beschränkt werden. Bevorzugt ist Cer-3-chlorid, das sowohl wasserfrei als auch als Hydrat oder Lösung verwendet werden kann.

Geeignete Lösungsmittel besitzen eine ausreichende Löslichkeit für die Reaktionspartner und reagieren im gewählten Temperaturbereich nicht mit ihnen. Bevorzugt ist Methanol; weiter können für sich oder im Gemisch verwendet werden: Ethanol, Tetrahydrofuran, Dimethylformamid und andere, gegebenenfalls unter Zusatz von Wasser.

Niedrige Temperaturen (-100 bis 0 °C) fördern die Bildung von 15α-Hydroxy-Isomeren. Die Reaktionszeiten betragen je nach Wahl der Reaktionsbedingungen zwischen wenigen Minuten bis zu einigen Stunden.

Die Herstellung von 1a und 3a sowie die Umsetzung der Verbindungen 2a, 4a und 5 zu Carbacyclin-Analogen ist bekannte (1a, 2a: Europäische Patentanmeldung 119 949; 3a, 4a, 5: Europäische Patentschrift 11 591).

3b wird in Analogie zu bekannten Verfahren (Schema 2) aus (1S,2S,3R,5R)-3-tert.-Butyldimethylsilyloxy-7,7-(2,2-dimethyltrimethylendioxy)-2-hydroxy-methyl-bicyclo[3.3.0]octan (6) hergestellt (Beispiel A1 und A2). Im Reduktionsprodukt 4b lassen sich durch Säurebehandlung die Schutzgruppen unter Bildung des bereits bekannten 5 entfernen.

Schema 2

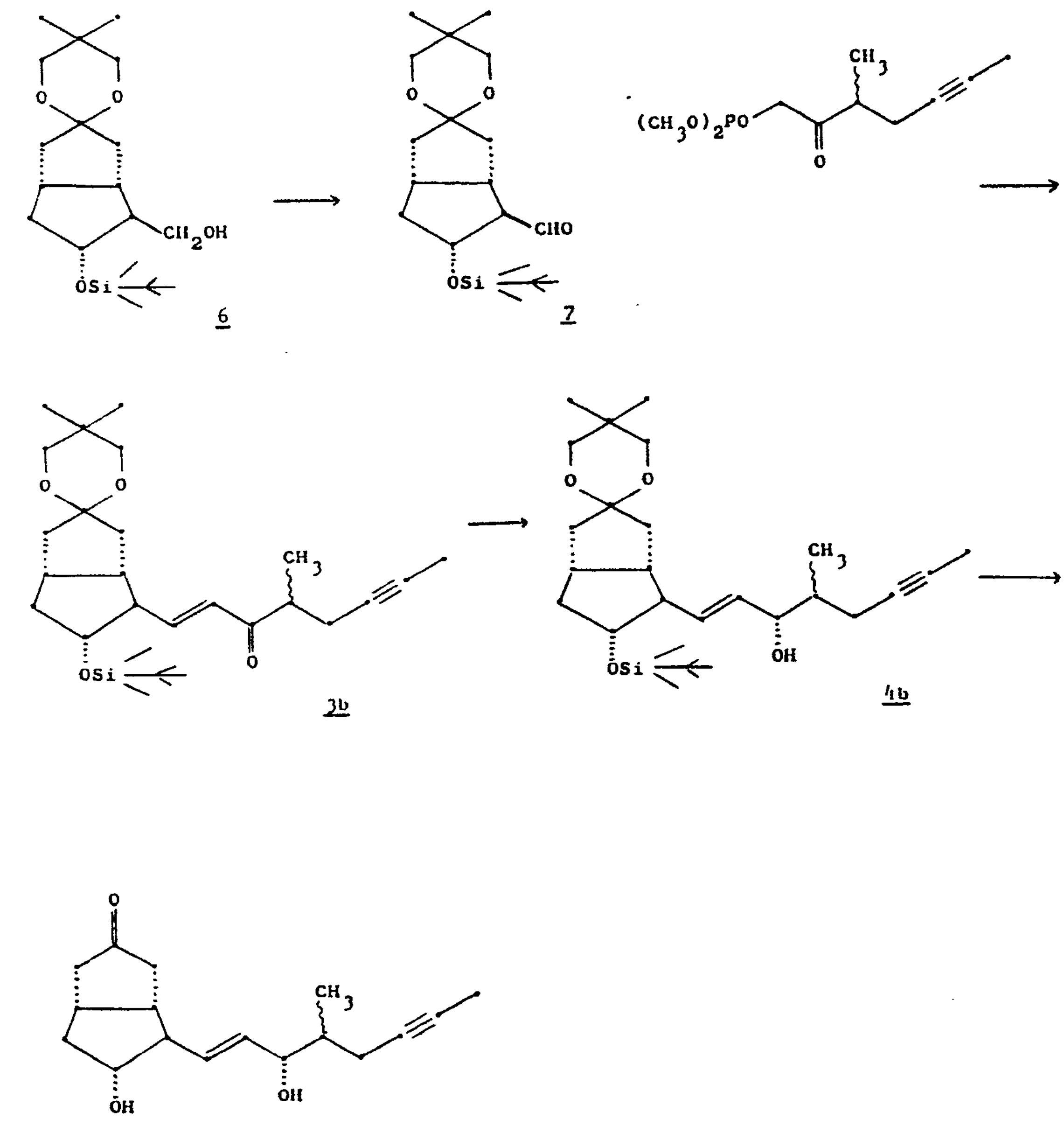

6 7 3b 4b

5

Das Verfahren läßt sich auch auf die durch die Formeln 8 und 9 repräsentierte Reihe mit der natürlich konfigurierten unteren Kette ausdehnen (Beispiel 7).

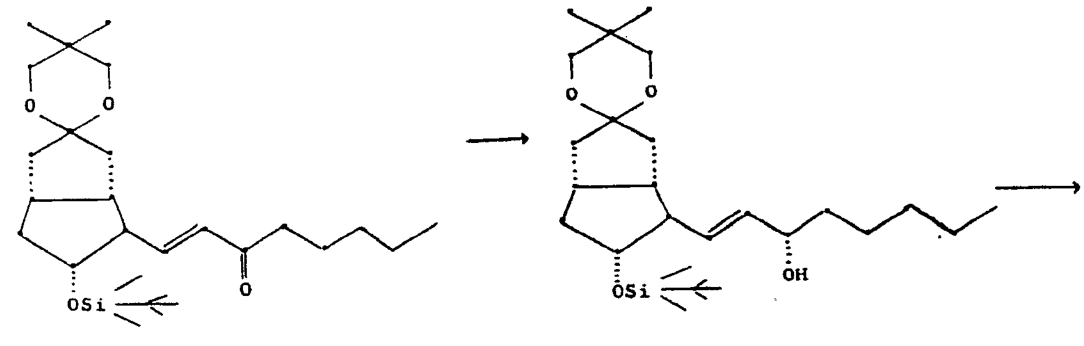

8 9

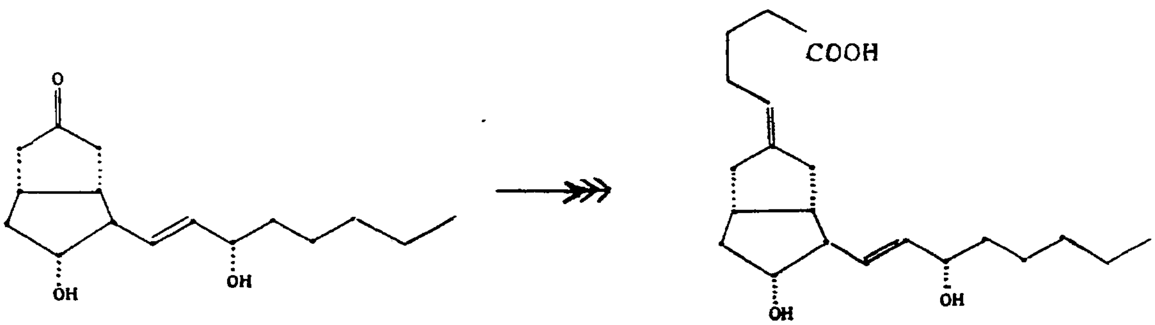

10 11

Die Herstellung des Ausgangsmaterials 8 erfolgt gemäß Beispiel A 4.

Die Verwendung des Reduktionsprodukts 9 zur Synthese von unsubstituiertem Carbacyclin 11 erfolgt durch Schutzgruppenabspaltung gemäß Beispiel A 5 zur Verbindung 10. Die Herstellung von Carbacyclin aus 10 wurde bereits von Kojima et al. Chem. Pharm. Bull. 33, 2588 (1985) beschrieben.

Wenn X einen geradkettigen oder verzweigtkettigen Alkylenrest mit 1 - 7 C-Atomen bedeutet, so sind damit folgende Reste gemeint:

$-(CH_2)_n-$ mit n = 1 - 7 (Methylen, Ethylen, Tri-, Tetra-, Penta-, Hexa-und Heptamethylen), $-C(CH_3)_2-$, $-CH-(CH_3)-$, $-CH(CH_3)-CH_2-$, $-C(CH_3)_2-CH_2-$, $-CH_2-CH(CH_3)-$, $CH_2-C(CH_3)_2-$, $-CH_2-CH(CH_3)-CH_2-$, $-CH_2-C(CH_3)_2-CH_2-$, $-CH(C_2H_5)-$, $-C(C_2H_5)_2-$, $-CH(C_2H_5)-CH_2-$, $-C(C_2H_5)_2-CH_2-$, $-CH_2-CH(C_2H_5)-$, $CH_2-C(C_2H_5)_2-$, $-CH_2-CH(C_2H_5)-CH_2-$, $-CH_2-C(C_2H_5)_2-$ usw.

Unter R, $R_1$, $R_2$ und $R_3$ als $C_1$-$C_7$-Alkyl werden Methyl, Ethyl, n-Propyl, Isopropyl, n-Butyl, Isobutyl, sek.-Butyl, tert.-Butyl, n-Pentyl, Isopentyl, sek.-Pentyl, Neopentyl, n-Hexyl, Isohexyl, Heptyl usw. verstanden.

D als Alkylgruppe mit 1 - 10 C-Atomen bedeutet außer den vorgenannten Alkylresten noch Octyl, Nonyl, Decyl und die dazugehörigen verzweigten Isomeren.

D als Alkenylgruppe mit 2 - 10 C-Atomen bedeutet bevorzugt

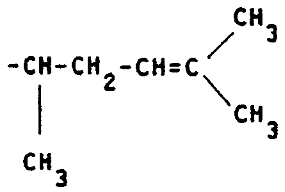, 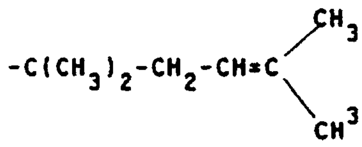, 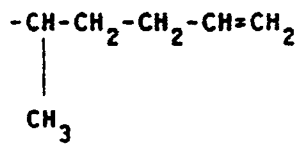

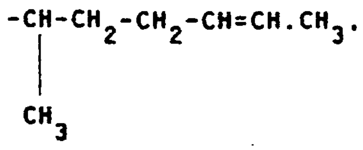 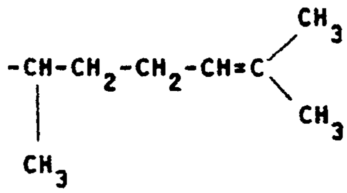

D als Alkinylgruppe mit 1 - 10 C-Atomen bedeutet zum Beispiel

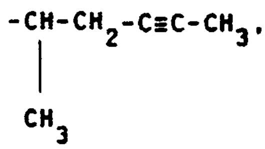 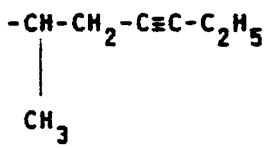 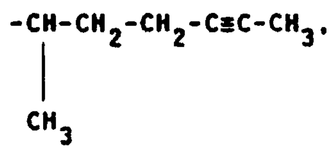

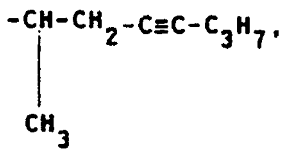 $-C(CH_3)_2-CH_2-C\equiv C-CH_3$ usw.

Die folgenden Ausführungsbeispiele sollen die Erfindung näher erläutern:

**Beispiel A 1**

(1S,,2R,3R,5R)-7,7-(2,2-Dimethyl-trimethylendioxy)-3-tert.butyldimethylsilyloxy -bicyclo[3.3.0]octan-2-carbaldehyd

Man löst 1.38 g Oxalylchlorid in 20 ml Dichlormethan, kühlt auf -60 °C ab und versetzt mit 1.87 g Dimethylsulfoxid in 6 ml Dichlormethan. Nach 10 Minuten fügt man eine Lösung von 2,886 g (-)-(1S,2S,3R,5R)-7,7-(2,2-Dimethyltrimethylendioxy)-3-tert.butyldimethylsilyloxy-2-hydroxymethyl-bicyclo-[3.3.0]octan in 13 ml Dichlormethan zu und rührt 30 Minuten. Dann tropft man 2,42 g Triethylamin in 5 ml Dichlormethan dazu. Nach 2 Stunden läßt man auf 0 °C erwärmen, fügt 260 ml Eiswasser zu, trennt die organische Phase ab, wäscht sie mit Natriumchlorid-Lösung. Nach Trocknen mit Natriumsulfat und Entfernen des Lösungsmittels im Vakuum erhält man ca. 3.0 g der Titelverbindung als Rohprodukt, das ohne weitere Reinigung verwendet werden kann.

**Beispiel A 2**

(1S,2S,3R,5R)-7,7-(2,2-Dimethyl-trimethylendioxy)-3-tert.butyldimethylsilyloxy-2-[(4R,S)(1E)-4-methyl-3-oxo-oct-1-en-6-inyl]bicyclo[3.3.0]octan

Man suspendiert 0,447 g Natriumhydrid (55 %) in 39 ml Tetrahydrofuran, kühlt im Eisbad und versetzt mit 2,58 g racemischem 3-Methyl-2-oxo-hept-5-in-yl-phosphonsäuredimethylester öin 20 ml Tetrahydrofuran. Man rührt 20 Minuten und gibt dann die 3.0 g des im Beispiel A 1 erhaltenen Carbaldehyds in 39 ml Tetrahydrofuran dazu. Nach 3 Stunden bei Eisbadtemperatur und 45 Minuten bei Raumtemperatur neutralisiert man mit Essigsäure, konzentriert im Vakuum, nimmt in Dichlormethan auf, wäscht mit Natriumhydrogencarbonat und Natriumchlorid-Lösung, trocknet mit Natriumsulfat, entfernt das Lösungsmittel und chromatographiert den Rückstand an Kieselgel mit Hexan-Ethylacetat-Gemischen. Man erhält 3,68 g Produkt mit $[\alpha]_D$ + 1,0°, $[\alpha]_{365}$ +26,6° ($CHCl_3$. c=1), das zur weiteren Umsetzung geeignet ist.

**Beispiel A 3**

(1S,2S,3R,5R)-3-Hydroxy-2-[(3S,4RS)(E)-3-hydroxy-4-methyl-oct-1-en-6-inyl]-bicyclo[3.3.0]octan-7-on

Man löst 2,278 g des gemäß Beispiel 3a erhaltenen unpolaren Produkts in 9 ml Tetrahydrofuran, 32,5 ml Essigsäure und 17,5 ml Wasser und erwärmt für 4 Stunden auf 45 °C. Dann destilliert man im Vakuum ab. zum Schluß unter Toluol-Zusatz, nimmt in Dichlormethan auf, extrahiert mit Wasser, trocknet mit Natriumsulfat, konzentriert im Vakuum und chromatographiert an Kieselgel mit Hexan-Ethylacetat-Gemischen. Man erhält 1,26 g der Titelverbindung, die chromatographisch und spektroskopisch identisch ist mit Material nach dem früher beschriebenen Syntheseweg, die für die Herstellung von Iloprost erforderliche Zusammensetzung der 16-Diastereomeren aufweist und dessen Enantiomerenreinheit (bestimmt durch HPLC der MTPA-Ester) größer als 99 % ist.

**Beispiel A 4**

(1S,2S,3R,5R)-7,7-(2,2-Dimethyl-trimethylendioxy)-3-tert.-butyldimethylsilyloxy-2-[(1E)-3-oxo-oct-1-enyl]-bicyclo-[3.3.0]octan

Man suspendiert 113 mg Natriumhydrid (55 %) in 10 ml Tetrahydrofuran und versetzt bei 20 °C mit 630 mg 2-Oxo-heptyl-phosphonsäure-dimethylester in 4,5 ml Tetrahydrofuran. Man rührt 30 Minuten und gibt dann 1,0 g des wie in Beispiel A 1 erhaltenen Carbaldehyds in 9 ml Tetrahydrofuran dazu. Nach 5 Stunden neutralisiert man mit Essigsäure, konzentriert im Vakuum, nimmt in Dichlormethan auf, wäscht mit Natriumchlorid-Lösung, trocknet mit Natriumsulfat, entfernt das Lösungsmittel und chromatographiert den Rückstand an Kieselgel mit Hexan/tert.-Butyl-methylether-Gemischen. Man erhält 1,19 g Produkt mit $[\alpha]D$ + 2,3° (Chloroform, c=1).

**Beispiel A 5**

(1S,2S,3R,5R)-3-Hydroxy-2-[(3S) (E)-3-hydroxy-oct-1-enyl]-bicyclo[3.3.0] octan -7-on

Man löst 0,54 g des gemäß Beispiel 7 erhaltenen unpolaren Produkts in 9 ml Ethanol, versetzt mit 6 ml Wasser und 0,06 ml konz. Salzsäure und rührt für 3 Stunden bei Raumtemperatur. Dann neutralisiert man mit Natriumhydrogencarbonat, destilliert im Vakuum ab, nimmt in Ethylacetat auf, extrahiert mit Natriumchlorid-Lösung, trocknet mit Natriumsulfat, konzentriert im Vakuum und chromatographiert an Kieselgel mit Hexan/Ethylacetat-Gemischen. Man erhält 0.27 g der Titelverbindung mit $[\alpha]D$ -11,2° (Methanol, c=1). Kojima et al (l. c.) geben -11,5° (Methanol, c=1) an.

**Beispiel 1**

(1S,2S,3R,5R)-2-[(1Z)(3S,4S)-2-Brom-3-hydroxy-4-methyl-non-1-en-6-inyl]-7,7-ethylendioxy-3-benzoyloxy-bicyclo[3.3.0]-octan

a) Erfindungsgemäß

Man löst 107,53 g (1S,2S,3R,5R)-2-[(1Z)(4S)-2-Brom-4-methyl-3-oxo-non-1-en-6-inyl]-7,7-ethylendioxy-3-benzoyloxy-bicyclo[3.3.0]-octan in 2 l Methanol, kühlt auf -40 °C ab, versetzt mit 11,68 g Cer-3-chlorid-heptahydrat, rührt 15 Minuten, trägt dann 12,37 g Natriumborhydrid in Portionen ein, rührt 30 Minuten, tropft Aceton im Überschuß dazu, ruhrt weitere 30 Minuten, neutralisiert mit Essigsäure, erwärmt und destilliert im Vakuum ab. Man löst den Rückstand in Dichlormethan und Wasser, wäscht mit Wasser, trocknet mit Natriumsulfat, konzentriert im Vakuum und chromatographiert an Kieselgel mit Dichlormethan/Ethylacetat-Gemischen.

Man erhält 55,2 g der Titelverbindung als unpolares Isomeres neben 47,0 g des polareren 3'R-Isomeren ($15\alpha:15\beta$ = 54:46).

b) Vergleichsansatz

Man führt die Umsetzunög wie unter a) beschrieben, jedoch ohne Cer-3-chlorid, durch. Um vollständige Umsetzung zu erzielen, muß die Natriumborhydrid-Menge erhöht werden. Man erhält die Isomeren im

Verhältnis 40:60.

**Beispiel 2**

(1S,2S,3R,5R)-2-[(Z)(3S,4S)-2-Brom-3-hydroxy-4-methyl-non-1-en-6-inyl]-7,7-(2,2-dimethyl-trimethylendioxy)-3-(4-phenylbenzoyloxy)-bicyclo[3.3.0]octan

a) Erfindungsgemäß

Man setzt 56,5 g (1S,2S,3R,5R)-2-[(Z)(4S)2-Brom-4-methyl-3-oxo non-1-en-6-inyl]-7,7-(2,2-dimethyl-trimethylendioxy]-3-(4-phenylbenzoyloxy)-bicyclo[3.3.0]octan nach den Bedingungen von Beispiel 1 mit 5,28 g Natriumborhydrid in Gegenwart von 4,96 g Cer-3-chlorid heptahydrat um und erhält 30,06 g der unpolareren 3'S-Verbindung neben 23,62 g der polareren 3'R-Verbindung ($15\alpha:15\beta$ = 56:44).

b) Vergleichsansatz

Man führt die Umsetzung wie unter a) beschrieben, jedoch ohne Cer-3-chlorid und mdit erhöhter Natriumborhydrid-Menge durch. Man erhält die Isomeren im Verhältnis $15\alpha:15\beta$ = 46:54.

**Beispiel 3**

(1S,2S,3R,5R)-7,7-(2,2-Dimethyl-trimethylendioxy)-3-tert.-butyldimethylsilyloxy-2-[(3S,4RS)(1E)-4-methyl-3-hydroxy-oct-1-en-6-inyl]bicyclo[3.3.0]-octan

a) Erfindungsgemäß

3,50 g des Ketons aus Beispiel A 2 löst man in 100 ml Methanol und kühlt auf -75 °C. Man gibt 2,76 g Cer-3-chlorid-heptahydrat zu, rührt 1 Stunde, versetzt mit 0,51 g Natriumborhydrid und rührt weitere 45 Minuten bei -75 °C. Nach Zugabe von Aceton wird langsam erwärmt, mit Essigsäure neutralisiert und im Vakuum konzentriert. Man löst den Rückstand in Dichlormethan, extrahiert mit Wasser, trocknet mit Natriumsulfat uönd konzentriert im Vakuum. Man chromatographiert an Kieselgel mit Hexan/tert.-Butylmethylether-Gemischen und erhält 2,52 g der Titelverbindung [unpolares Isomeres, $[\alpha]_D$ + 8,8°, $[\alpha]_{365}$ + 24.2° ($CHCl_3$. c=1)] und 0,25 g des polareren 3'R-Isomeren.

HPLC-Messungen zeigen, daß in der Titelverbindung die Methylisomeren im Verhältnis $\alpha:\beta$ = 54:46 vorliegen.

b) Vergleichsansatz

Man führt die Umsetzung wie unter a) beschrieben, jedoch ohne Cer-3-chlorid und mit erhöhter Natriumborhydrid-Menge durch. Man erhält die Isomeren im Verhältnis 3'S:3'R = 74:26.

Im 3'S-Anteil liegen die Methylisomeren im Verhältnis $\alpha:\beta$ = 59:41 vor.

**Beispiel 4**

(1S,2S,3R,5R)-2-[(1Z) (3S,4S)-2-Brom-3-hydroxy-4-methyl-non-1-en-6-inyl]-7,7-(2,2-dimethyl-trimethylen-dioxy)-3-(-tert.-butyl-dimethylsilyloxy-bicyclo[3.3.0]octan

Man reduziert 106,0 g (1S,2S,3R,5R-2-[(1Z) (4S)-2-Brom-4-methyl-3-oxo-non-1-en-6-inyl]-7,7-(2,2-dimethyl-trimethylen-dioxy)-3-(-tert.-butyl-dimethyl-silyloxy-bicyclo[3.3.0]octan gemäß Beispiel 3. Im Rohprodukt liegen die $15\alpha/15\beta$-Isomeren nach HPLC im Verhältnis 88,9 : 11,1 vor. Bei der Auftrennung des Gemisches durch Chromatographie erhält man 92.2 g des unpolareren $15\alpha$-Hydroxy-Isomeren $[\alpha]D$ + 21° (Chloroform, c=1) und 8.0 g des polareren $15\beta$-Hydroxy-Isomeren.

**Beispiel 5**

(1S,2S,3R,5R)-7,7-(2,2-Dimethyl-trimethylendioxy)-3-(2,3-dimethyl-but-2-yl)-dimethyl-silyloxy-2-[(3S,4RS) (1E)-4-methyl-3-hydroxy-oct-1-en-6-inyl]bicyclo[3.3.0]octan

4,5 g (1S,2S,3R,5R)-7,7-(2,2-Dimethyl-trimethylendioxy)-3-(2,3-di-methyl-but-2 -yl)-dimethyl-silyloxy-2[-(4RS) (1E)-4-methyl-3-oxo-oct-1-en-6-inyl]bicyclo[3.3.0]octan werden gemäß Beispiel 3a) reduziert. Man erhält 3,16 g des unpolaren $15\alpha$-Hydroxy-Isomeren $[\alpha]D$ +3,0° (Chloroform, c=1). 0,65 g des polaren $15\beta$-Hydroxy-Isomeren und 0,57 g Mischfraktionen.

HPLC-Messungen nach Abspaltung der Schutzgruppen zeigen, daß die Methylisomeren im Verhältnis $\alpha:\beta$ = 54:46 vorliegen.

**Beispiel 6**

(1S,2S,3R,5R)-7,7-(2,2-Dimethyl-trimethylendioxy)-3-tert.-butyl-diphenyl-silyloxy-2-[(3S,4RS) (1E)-4-methyl-3-hydroxy-oct-1-en-6-inyl]bicyclo[3.3.0]octan

3,0 g (1S,2S,3R,5R)-7,7-(2,2-Dimethyl-trimethylendioxy)-3-tert.-butyl-diphenyl-silyloxy-2-[(4RS) (1E)-4-methyl-3-oxo-oct-1-en-6-inyl]bicyclo[3.3.0]octan werden gemäß Beispiel 3a) reduziert. Man erhält 2,60 g des unpolaren 15α-Hydroxy-Isomeren [α]D +21,1° (Chloroform, c=1) und 0,35 g des polaren 15β-Hydroxy-Isomeren, das noch etwas 15α-Hydroxy-Isomeres enthält.

HPLC-Mesungen nach Abspaltung der Schutzgruppen zeigen, daß die Methylisomeren im Verhältnis α:β = 53:47 vorliegen.

**Beispiel 7**

(1S,2S,3R,5R)-7,7-(2,2-Dimethyl-trimethylendioxy)-3-tert.-butyldimethylsilyloxy-2-[(1E) (3S)-3-hydroxy-oct-1-enyl]-bicyclo[3.3.0]octan

10, 0 g des in Beispiel A 4 erhaltenen Ketons werden gemäß Beispiel 3a) reduziert. Man erhält 8,05 g der unpolaren 15α-Hydroxy-Verbindung mit [α]D -3,5° (Chloroform, c=1) neben 1,30 g des polaren 15β-Hydroxy-Isomeren.

**Patentansprüche**

1. Verfahren zur Herstelldung von α-Hydroxy-bicyclo[3.3.0]octan-Derivaten der Formel I

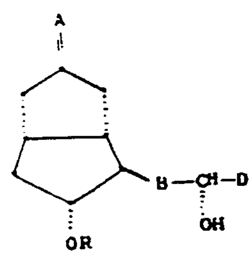

(I),

worin

A den 2bindigen Rest -O-X-O- mit X als gerad- oder verzweigtkettigem Alkylen mit 1 - 7 C-Atomen oder die Reste
$=CH-(CH_2)_3-COOR'$, $=CH-CH_2-O-CH_2-COOR'$ oder
$=CH-(CH_2)_3-O-CH_2-CH_2-COOR'$ mit R' als $C_1$-$C_7$-Alkyl bedeutet,

R den Rest

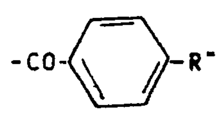

mit R" als Wasserstoff oder Phenyl oder den Rest $-SiR_1R_2R_3$, wobei $R_1$,$R_2$ und $R_3$ gleich oder verschieden sein können und eine gerad- oder verzweigtkettige Alkylgruppe mit 1 - 7 C-Atomen oder Phenyl darstellen.

B eine trans-CH=C(X)-Gruppe mit X als Wasserstoff oder Brom, wobei sich die trans-Konfiguration auf die C-Kette bezieht, und

D eine Alkylgruppe mit 1 - 10 C-Atomen, eine Alkenylgruppe mit 2 - 10 C-Atomen oder eine Alkinylgruppe mit 2-10 C-Atomen bedeuten,

dadurch gekennzeichnet, daß man Keto-bicyclo[3.3.0]octan-Derivate der Formel II

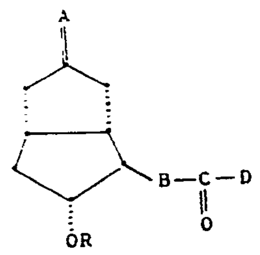

(II),

in Gegenwart von Cer-III-Salzen reduziert.

2. Verfahren nach Anspruch 1, dadurch gekennzeichnet, daß man als Reduktionsmittel $NaBH_4$ verwendet.

3. Verfahren nach Anspruch 1, dadurch gekennzeichnet, daß man als Cer-III-Salz Cer-III-Chlorid verwendet.

4. Verfahren nach Anspruch 1, dadurch gekennzeichnet, daß man als Ausgangsverbindung ein Keto-bicyclo[3.3.0]octan-Derivat der Formel II einsetzt, worin R einen $R_3R_2R_1Si$-Rest mit $R_1$, $R_2$ und $R_3$ in den oben angegebenen Bedeutungen darstellt.

## Claims

1. Process for the preparation of α-hydroxy-bicyclo[3.3.0]octane derivatives of formula I

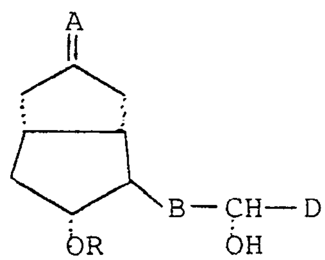

(I)

in which

A represents the divalent radical -O-X-O- wherein X is straight-chained or branched alkylene having from 1 to 7 carbon atoms, or represents a radical
$=CH-(CH_2)_3-COOR'$, $=CH-CH_2-O-CH_2-COOR'$ or
$=CH-(CH_2)_3-O-CH_2-CH_2-COOR'$ wherein R' is $C_1$-$C_7$-alkyl,

R represents the radical

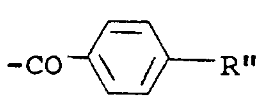

wherein R" is hydrogen or phenyl, or represents the radical $-SiR_1R_2R_3$ wherein $R_1$, $R_2$ and $R_3$ may be identical or different and represent a straight-chained or branched alkyl group having from 1 to 7 carbon atoms or phenyl,

B represents a trans-CH=C(X) group wherein X is hydrogen or bromine, the trans-configuration relating to the C-chain, and

D represents an alkyl group having from 1 to 10 carbon atoms, an alkenyl group having from 2 to 10 carbon atoms or an alkynyl group having from 2 to 10 carbon atoms,

characterised in that keto-bicyclo[3.3.0]octane derivatives of formula II

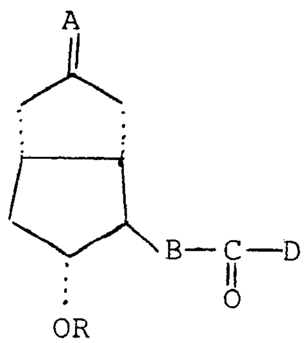

(II)

are reduced in the presence of Cer-III salts.

2. Process according to claim 1, characterised in that $NaBH_4$ is used as reducing agent.

3. Process according to claim 1, characterised in that Cer-III chloride is used as Cer-III salt.

4. Process according to claim 1, characterised in that there is used as the starting compound a keto-bicyclo[3.3.0]octane derivative of formula II in which R represents an $R_3R_2R_1Si$ radical wherein $R_1$, $R_2$ and $R_3$ have the meanings given above.

**Revendications**

1. Procédé pour préparer des α-hydroxy-bicyclo[3.3.0]octanes répondant à la formule I :

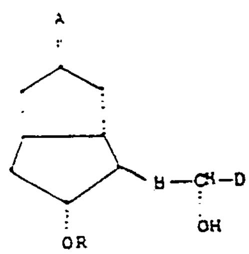

(I)

dans laquelle

A représente le radical à deux liaisons -O-X-O- dont le symbole X représente un alkylène linéaire ou ramifié qui contient de 1 à 7 atomes de carbone, ou l'un des radicaux :

$=CH-(CH_2)_3-COOR'$ ,
$=CH-CH_2-O-CH_2-COOR'$ et
$=CH-(CH_2)_3-O-CH_2-CH_2-COOR'$

dans lesquels R' représente un alkyle en $C_1$-$C_7$,

R représente :

- un radical

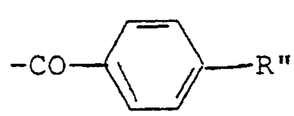

dans lequel R" représente l'hydrogène ou un phényle, ou
- un radical $-SiR_1R_2R_3$ dans lequel $R_1$, $R_2$ et $R_3$ peuvent représenter chacun, indépen-

damment les uns des autres, un radical alkyle, linéaire ou ramifié, contenant de 1 à 7 atomes de carbone ou un radical phényle,

B représente un radical -CH=C(X)- trans dans lequel X représente l'hydrogène ou le brome, la configuration trans concernant la chaîne carbonée, et

D représente un radical alkyle contenant de 1 à 10 atomes de carbone, un radical alcényle contenant de 2 à 10 atomes de carbone ou un radical alcynyle contenant de 2 à 10 atomes de carbone,

procédé caractérisé en ce qu'on réduit des oxobicyclo[3.3.0]octanes de formule II :

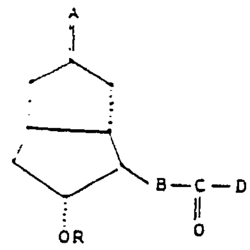

(II),

en présence de sels de cérium(III).

2. Procédé selon la revendication 1 caractérisé en ce qu on utilise NaBH4 comme réducteur.

3. Procédé selon la revendication 1 caractérisé en ce qu'on utilise, comme sel de cérium(III), le chlorure de cérium(III).

4. Procédé selon la revendication 1 caractérisé en ce qu'on utilise, comme corps de départ, un oxobicyclo[3.3.0]octane de formule II dans lequel R représente un radical $R_3R_2R_1Si$- dont les symboles $R_1$, $R_2$ et $R_3$ ont les significations qui leur ont été données ci-dessus.